(19) Europäisches Patentamt
European Patent Office
Office européen des brevets

(11) Veröffentlichungsnummer: **0 199 323**
**A2**

# (12) EUROPÄISCHE PATENTANMELDUNG

(21) Anmeldenummer: **86105468.2**

(22) Anmeldetag: **21.04.86**

(51) Int. Cl.⁴: **C07D 261/12**, C07D 413/06, A61K 31/42

(30) Priorität: **26.04.85 DE 3515093**

(43) Veröffentlichungstag der Anmeldung: **29.10.86 Patentblatt 86/44**

(84) Benannte Vertragsstaaten: **AT BE CH DE FR GB IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Kreutzberger, Alfred, Prof. Dr.**
**Wormser Strasse 171f**
**D-6500 Mainz(DE)**
Erfinder: **Kolter, Karl, Dr.**
**Am Linsenberg 21**
**D-6500 Mainz(DE)**
Erfinder: **Below, Peter, Dr.**
**Geisenheimer Strasse 88**
**D-6000 Frankfurt am Main 71(DE)**
Erfinder: **Hock, Franz, Dr.**
**Altstadt 19**
**D-6110 Dieburg(DE)**

## (54) 4-Aminoalkylidensubstituierte 3-Aryl-5(4H)-isoxazolone, Verfahren zur ihrer Herstellung, diese Verbindungen enthaltende Arzneimittel und ihre Verwendung.

(57) 4-Aminoalkylidensubstituierte 3-Aryl-5(4H)-isoxazolone der Formel I

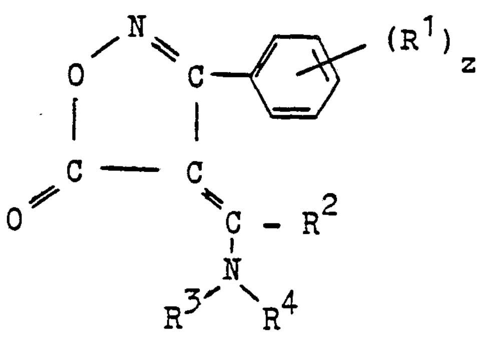

(I)

worin R¹, R², R³, R⁴ und z die angegebenen Bedeutungen haben, Verfahren zu ihrer Herstellung und pharmazeutische Präparte auf Basis dieser Verbindungen werden beschrieben. Sie können zur Behandlung cognitiver Dysfunktionen verwendet werden

EP 0 199 323 A2

# 4-Aminoalkylidensubstituierte 3-Aryl-5(4H)-isoxazolone, Verfahren zu ihrer Herstellung, diese Verbindungen enthaltende Arzneimittel und ihre Verwendung

Die Erfindung betrifft neue 4-Aminoalkyliden-substituierte 5(4H)-Isoxazolone, die als solche oder in Form ihrer pharmakologisch verträglichen Salze nootrope (die cognitiven Funktionen verbessernde) Wirkung aufweisen, Verfahren zur Herstellung dieser Verbindungen, pharmazeutische Mittel, welche die erfindungsgemäßen aktiven Verbindungen enthalten und deren Verwendung als Arzneimittel, insbesondere zur Behandlung cognitiver Dysfunktionen.

3-Phenyl-4-(1-aminoalkyliden)-5(4H)-isoxazolone und 3-Phenyl-4-(1-aminobenzyliden)-5-(4H)-isoxazolone wurden bereits beschrieben (z.B. R. Stolle, Z. Chem. 20(1), 20 -21 (1980), O.S. Wolfbeis, Monatsh. Chem. 112(3), 369 -383 (1981) und W. Müller et al., Tetrahedron 29, 4291 -4298 - (1973)). Über pharmakologische Wirkungen dieser Verbindungen ist nichts bekannt.

Es wurde nun überraschenderweise gefunden, daß 5(4H)-Isoxazolone der allgemeinen Formel I

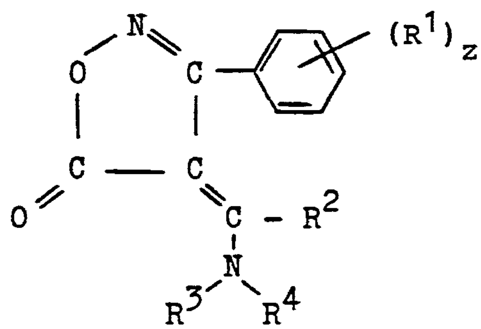

(I)

worin

$R^1$ Wasserstoff, $C_1$-$C_4$-Alkyl, $C_1$-$C_4$-Alkoxy, Hydroxy, Halogen, Nitro oder Trifluormethyl ist,

z für 1, 2 oder 3 steht,

$R^2$ Wasserstoff oder $C_1$-$C_4$-Alkyl bedeutet,

$R^3$ Wasserstoff oder $C_1$-$C_4$-Alkyl und

$R^4$ Wasserstoff, $C_1$-$C_{10}$-Alkyl, vorzugsweise $C_1$-$C_4$-Alkyl, oder eineGruppe der Formel $-(CH_2)_n-R^5$ ist, worin n = 1 bis 10, vorzugsweise 1 bis 5, ist und $R^5$ ein Phenylrest, der mit $C_1$-$C_4$-Alkyl, $C_1$-$C_4$-Alkoxy, Halogen, Nitro oder Trifluormethyl substituiert sein kann, oder ein Aminorest der Formel

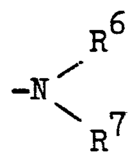

ist, worin $R^6$ Wasserstoff oder $C_1$-$C_4$-Alkyl und $R^7$ $C_1$-$C_4$-Alkyl oder Phenyl-($C_1$-$C_4$)alkyl bedeutet oder worin $R^6$ und $R^7$ zusammen eine Alkylenkette mit 3 bis 7 Methylengruppen darstellen, die gegebenenfalls substituiert ist mit $C_1$-$C_4$-Alkyl oder Phenyl, das gegebenenfalls substituiert ist mit $C_1$-$C_4$-Alkyl, $C_1$-$C_4$-Alkoxy, Halogen, Nitro oder Trifluormethyl, oder in der eine $CH_2$-Gruppe gegebenenfalls ersetzt ist durch O, S oder N-$R^8$, wobei $R^8$ Wasserstoff, $C_1$-$C_4$-Alkyl oder Phenyl, das gegebenenfalls mit $C_1$-$C_4$-Alkyl, $C_1$-$C_4$-Alkoxy, Halogen, Nitro oder Trifluormethyl substituiert ist, darstellt oder $R^3$ und $R^4$ bilden zusammen mit dem Stickstoffatom einen gesättigten oder ungesättigten mono-oder bicyclischen Ring mit insgesamt 4 bis 10 Ringgliedern, der außer dem Stickstoffatom als zweites Heteroatom ein Sauerstoff-, Schwefel-oder Stickstoffatom aufweisen kann und der gegebenenfalls substituiert ist mit $C_1$-$C_4$-Alkyl, vorzugsweise Methyl oder Ethyl, mit $C_1$-$C_4$-Alkoxycarbonyl, vorzugsweise $C_1$-$C_2$-Alkoxycarbonyl, mit Carbamoyl, mit Cinnamyl, mit Phenyl, das mit Halogen, $C_1$-$C_4$-Alkyl, vorzugsweise Methyl, $C_1$-$C_4$-Alkoxy, vorzugsweise Methoxy oder Trifluormethyl substituiert sein kann, oder mit der Gruppe $(CH_2)_y$ $-CH_{3-m}R^9m$, worin y = 0 bis 3, m 1 oder 2 und $R^9$ ein Phenylrest ist, der mit Halogen, $C_1$-$C_4$-Alkyl, vorzugsweise Methyl, $C_1$-$C_4$-Alkoxy, vorzugsweise Methoxy, oder Trifluormethyl substituiert sein kann, wobei Verbindungen, worin $R^1$ und $R^2$ Wasserstoff und $R^3$ und $R^4$ Methyl bedeuten, ausgenommen sind, wertvolle pharmazeutische Eigenschaften besitzen.

Die Erfindung betrifft daher 5(4H)-Isoxazolone der allgemeinen Formel I sowie deren pharmakologisch verträgliche Säureadditionssalze.

In den vorstehenden und folgenden Ausführungen wird unter Alkyl geradkettiges und verzweigtes Alkyl verstanden. Halogen bedeutet vorzugsweise Chlor.

$R^1$ ist vorzugsweise Wasserstoff, Chlor oder Methoxy und z = 1. $R^2$ bedeutet insbesondere Wasserstoff.

Falls $R^4$ einen Rest $-(CH_2)_n-R^5$ darstellt, bedeutet $R^5$ vorzugsweise einen cyclischen tertiären Aminorest wie z.B. Azetidino, Pyrrolidino, Piperidino, Morpholino, Piperazino, Perhydroazepino, Homopiperazino, wobei diese Ringe, insbesondere wenn es sich um Piperidin-, Piperazin-oder Homopiperazinringe handelt, noch substituiert sein können mit $C_1$-$C_4$-Alkyl, Phenyl oder Phenyl, das mit $C_1$-$C_2$-Alkyl, $C_1$-$C_2$-Alkoxy, Chlor, Nitro oder Trifluormethyl substituiert ist.

$R^3$ und $R^4$ können gemeinsam ein heterocyclisches Ringsystem bilden. Als in Betracht kommende Ringe seien genannt der Azetidin-, Pyrrolidin-, Piperidin-, 1,2,3,6-Tetrahydropyridin-, Perhydroazepin-, Perhydroazocin-, Piperazin-, Homopiperazin-, Morpholin-, Thiomorpholin-oder 1,2,3,4-Tetrahydroisochinolinring. Insbesondere wenn es sich um einen Piperidin-, 1,2,3,6-Tetrahydropyridin-, Piperazin-oder Homopiperazinring handelt, kann dieser noch mit Methyl, Ethyl, $C_1$-$C_2$-Alkoxycarbonyl, Carbamoyl, Cinnamyl oder Phenyl substituiert sein.

Ganz besonders bevorzugt sind Verbindungen der Formel I worin $R^1$ Wasserstoff oder Chlor und $R^2$ Wasserstoff bedeuten und $R^3$ und $R^4$ zusammen mit dem Stickstoffatom einen gesättigten, monocyclischen Ring mit 6 bis 8 Ringgliedern bilden, wobei dieser Ring vorzugsweise mit Phenyl substituiert sein kann.

Als geeignete, physiologisch verträgliche Säureadditionssalze seien genannt: Hydrochloride, Hydrobromide, Hydrojodide, Phosphate, Nitrate, Sulfate, Acetate, Citrate, Gluconate, Benzoate, Butyrate, Sulfosalicyclate, Maleate, Laurate, Malate, Fumarate, Succinate, Oxalate, Tartrate, Stearate, Methansulfonate und Toluolsulfonate.

Die Erfindung betrifft weiterhin Verfahren zur Herstellung der Verbindungen der allgemeinen Formel I, welche dadurch gekennzeichnet sind, daß man

a) eine Verbindung der allgemeinen Formel II

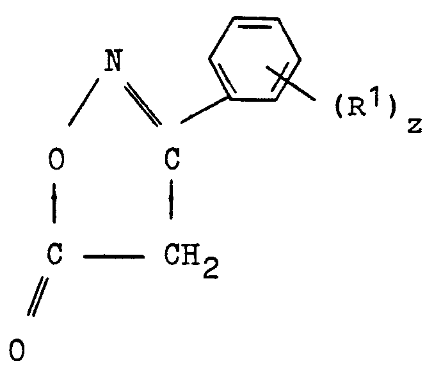

(II)

und ein Amin der Formel III

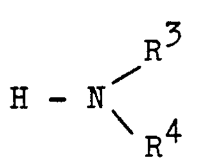

(III)

worin $R^1$, $R^3$, $R^4$ und z die gleichen Bedeutungen wie in Formel I haben, mit 1,3,5,-Triazin zu einer Verbindung der Formel I, worin $R^2$ Wasserstoff ist und $R^1$, $R^3$, $R^4$ und z die angegebenen Bedeutungen haben umsetzt;

b) eine Verbindung der allgemeinen Formel II und ein Amin der allgemeinen Formel III, worin z, $R^1$, $R^3$ und $R^4$ die gleichen Bedeutungen wie in Formel I haben, mit einem Orthoester der allgemeinen Formel IV,

$R^2C(OR^{10})_3$ (IV)

worin $R^2$ die oben genannte Bedeutung hat und $R^{10}$ Methyl oder Ethyl ist, umsetzt; oder

c) eine Verbindung der allgemeinen Formel V,

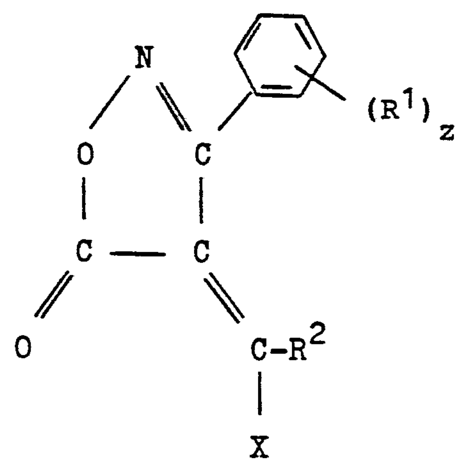

(V)

worin z, $R^1$ und $R^2$ die unter Formel I aufgeführte Bedeutung haben und X eine Hydroxy-, Methoxy, Ethoxy-, Amino-, $C_1$-$C_4$-Alkylamino, $C_1$-$C_4$-Dialkylamino-oder Anilinogruppe ist, mit einem Amin der allgemeinen Formel III umsetzt

und die Reaktionsprodukte gegebenenfalls in physiologisch verträgliche Säureadditionsalze überführt.

Die Herstellung der erfindungsgemäßen Verbindungen gemäß Formel I nach Verfahren a aus einer Verbindung der allgemeinen Formel II, einem Amin der Formel III und 1,3,5-Triazin wird bei einer Temperatur zwischen 0°C und dem Siedepunkt des verwendeten Lösungsmittels, vorzugsweise zwischen 20 une 75°C, durchgeführt. Geeignete Lösungsmittel sind inerte organische Lösungsmittel wie aliphatische, ali cyclische oder aromatische Kohlenwasserstoffe, insbesondere Petrolether, Cyclohexan oder Toluol, chlorierte Kohlenwasserstoffe, Ether wie Diethylether, Tetrahydrofuran, Dioxan, Dimethoxyethan oder Diglyme, oder Dimethylformamid, Dimethylacetamid, N-Methylpyrrolidon, Sulfolan oder Dimethylsulfoxyd. Bevorzugt werden chlorierte Kohlenwasserstoffe als Lösungsmittel, insbesondere Chloroform, Methylenchlorid oder Tetrachlorkohlenstoff. Vorteilhafterweise leitet man während der Reaktion einen Strom von trockenem Stickstoff, Argon oder Helium durch das Reaktionsgemisch.

Nach beendeter Umsetzung wird das Produkt, gegebenenfalls nach Abdampfen des verwendeten Lösungsmittels, abfiltriert und durch Umkristallisation oder Säulenchromatographie gereinigt.

In Verfahren b werden eine Verbindung der allgemeinen Formel II, ein Amin der Formel III und ein Orthoester der Formel IV miteinander umgesetzt. Dabei kann die Reaktion in Analogie zu dem von O.S. Wolfbeis beschriebenen Verfahren (Monatsh. Chem. 112(3), 369-83, (1981)) entweder ohne Lösungsmittel oder in einem organischen Lösungsmittel wie Eisessig, Dimethylsulfoxyd, Sulfolan oder einem Alkohol, bevorzugt einem $C_1$-$C_4$-Alkohol, insbesondere Isopropanol, Methanol, Ethanol, Propanol oder Butanol durchgeführt werden. Die Reaktion wird bei Temperaturen zwischen 0°C und dem Siedepunkt des verwendeten Lösungsmittels, insbesondere zwischen 50 und 80°C durchgeführt. Bei der Variante ohne Lösungsmittel ist es vorteilhaft, eine katalytische Menge Essigsäure zuzugeben und den gebildeten Alkohol abzudestillieren. Nach beendeter Umsetzung wird das Produkt durch Abkühlen auf 0 -20°C oder durch Zufügen von Wasser zur Kristallisation gebracht und durch Umkristallisieren gereinigt.

In Verfahren c wird eine Verbindung der allgemeinen Formel V mit einem Amin der Formel III umgesetzt. Die Reaktion wird je nach der Bedeutung von X in Analogie zu Verfahren von W. Müller et al., Tetrahedron 29(24), 4291-8 (1973), O. S. Wolfbeis, Monatsh. Chem. 112(3), 369-83, (1981), und G. Papini, Gazz. Chim. Ital. 81, 230, 1951)

vorteilhafterweise in einem organischen Lösungsmittel wie einem aromatischen Kohlenwasserstoff, insbesondere Toluol, Xylol oder Mesitylen, oder einem Alkohol, insbesondere einem $C_1$-$C_4$-Alkohol, vorteilhafterweise Isopropanol, Methanol, Ethanol, Propanol oder Butanol durchgeführt. Die Reaktion wird zweckmäßigerweise bei Temperaturen zwischen 20°C und dem Siedepunkt des verwendeten Lösungsmittels durchgeführt, wobei Temperaturen zwischen 50 und 100°C besonders vorteilhaft sind.

Nach beendeter Umsetzung wird das Produkt, gegebenenfalls nach Abdampfen des verwendeten Lösungsmittels, abfiltriert und durch Umkristallisation oder Säulenchromatographie gereinigt.

Die Amine der allgemeinen Formel III sind entweder in der Literatur beschrieben oder können nach literaturbekannten Methoden hergestellt werden.

Die Verbindungen der allgemeinen Formel II sind entweder in der Literatur beschrieben ($R^1$ = Wasserstoff) oder können nach dem Verfahren von Hantzsch (Ber. d. deut. chem. Ges. 24, 502 (1891)) aus den bekannten Benzoylessigestern der allgemeinen Formel VI,

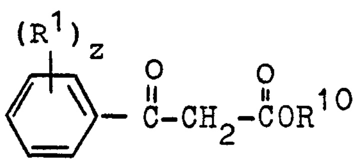

(VI)

in der $R^1$ und z die unter Formel I genannte Bedeutung hat und $R^{10}$ Methyl oder Ethyl ist, durch Umsetzung mit Hydroxylamin gewonnen werden.

Die Verbindungen der allgemeinen Formel V sind aus Verbindungen der allgemeinen Formel II durch Kondensation mit N,N'-Diphenylamidinen - (Mansberg, E., Shaw, G., J.Chem. Soc. 3467 - (1953)) oder Orthocarbonsäureestern (W. Müller et al., Tetrahedron 29(24), 4291-8 (1973)) erhältlich (X = OMe, OEt, NHPh); Verbindungen der Formel V mit X=OH sind aus solchen mit X= OMe, OEt oder NHPh durch Behandlung mit Alkali zugänglich (W. Müller et. al. loc.cit.).

Als erfindungsgemäße Verbindungen können außer den in den Beispielen genannten vorzugsweise die folgenden hergestellt werden:

4-Piperidinomethylen-3-(4-chlorphenyl)-2-isoxazolin-5-on

4-(2-Dimethylaminoethylamino)methylen-3-(4-chlorphenyl)-2-isoxazolin-5-on

4-Diethylaminomethylen-3-(4-chlorphenyl)-2-isoxazolin-5-on

4-Morpholinomethylen-3-(4-chlorphenyl)-2-isoxazolin-5-on

4-(4-Phenylpiperidin-1-yl)methylen-3-(4-chlorphenyl)-2-isoxazolin-5-on

4-Perhydroazocin-1-ylmethylen-3-(4-chlorphenyl)-2-isoxazolin-5-on

4-Piperidinomethylen-3-(3-chlorphenyl)-2-isoxazolin-5-on

4-(2-Dimethylaminoethylamino)methylen-3-(3-chlorphenyl)-2-isoxazolin-5-on

4-Diethylaminomethylen-3-(3-chlorphenyl)-2-isoxazolin-5-on

4-Morpholinomethylen-3-(3-chlorphenyl)-2-isoxazolin-5-on

4-(4-Phenylpiperidin-1-yl)methylen-3-(3-chlorphenyl)-2-isoxazolin-5-on

4-Perhydroazocin-1-ylmethylen-3-(3-chlorphenyl-2-isoxazolin-5-on

4-Piperidinomethylen-3-(2-chlorphenyl)-2-isoxazolin-5-on

4-(2-Dimethylaminoethylamino)methylen-3-(2-chlorphenyl)-2-isoxazolin-5-on

4-Diethylaminomethylen-3-(2-chlorphenyl)-2-isoxazolin-5-on

4-Morpholinomethylen-3-(2-chlorphenyl)-2-isoxazolin-5-on

4-(4-Phenylpiperidin-1-yl)methylen-3-(2-

chlorphenyl)-2-isoxazolin-5-on

4-Perhydroazocin-1-ylmethylen-3-(2-chlorphenyl)-2-isoxazolin-5-on

4-Piperidinomethylen-3-(4-methoxyphenyl)-2-isoxazolin-5-on

4-(2-Dimethylaminoethylamino)methylen-3-(4-methoxyphenyl)-2-isoxazolin-5-on

4-Diethylaminomethylen-3-(4-methoxyphenyl)-2-isoxazolin-5-on

4-Morpholinomethylen-3-(4-methoxyphenyl)-2-isoxazolin-5-on

4-(4-Phenylpiperidin-1-yl)methylen-3-(4-methoxyphenyl)-2-isoxazolin-5-on

4-Perhydroazocin-1-ylmethylen-3-(4-methoxyphenyl)-2-isoxazolin-5-on

4-Piperidinomethylen-3-(3-methoxyphenyl)-2-isoxazolin-5-on

4-(2-Dimethylaminoethylamino)methylen-3-(3-methoxyphenyl)-2-isoxazolin-5-on

4-Diethylaminomethylen-3-(3-methoxyphenyl)-2-isoxazolin-5-on

4-Morpholinomethylen-3-(3-methoxyphenyl)-2-isoxazolin-5-on

4-(4-Phenylpiperidin-1-yl)methylen-3-(3-methoxyphenyl)-2-isoxazolin-5-on

4-Perhydroazocin-1-ylmethylen-3-(3-methoxyphenyl)-2-isoxazolin-5-on

4-Piperidinomethylen-3-(2-methoxyphenyl)-2-isoxazolin-5-on

4-(2-Dimethylaminoethylamino)methylen-3-(2-methoxyphenyl)-2-isoxazolin-5-on

4-Diethylaminomethylen-3-(2-methoxyphenyl)-2-isoxazolin-5-on

4-Morpholinomethylen-3-(2-methoxyphenyl)-2-isoxazolin-5-on

4-(4-Phenylpiperidin-1-yl)methylen-3-(2-methoxyphenyl)-2-isoxazolin-5-on

4-Perhydroazocin-1-ylmethylen-3-(2-methoxyphenyl)-2-isoxazolin-5-on

4-Piperidinomethylen-3-(3,4-dimethoxyphenyl)-2-isoxazolin-5-on

4-(2-Dimethylaminoethylamino)methylen-3-(3,4-dimethoxyphenyl)-2-isoxazolin-5-on

4-Diethlaminomethylen-3-(3,4-dimethoxyphenyl)-2-isoxazolin-5-on

4-Morpholinomethylen-3-(3,4-dimethoxyphenyl)-2-isoxazolin-5-on

4-(4-Phenylpiperidin-1-yl)methylen-3-(3,4-dimethoxyphenyl)-2-isoxazolin-5-on

4-Perhydroazocin-1-ylmethylen-3-(3,4-dimethoxyphenyl)-2-isoxazolin-5-on

4-Piperidinomethylen-3-(3,4,5-trimethoxyphenyl)-2-isoxazolin-5-on

4-(2-Dimethylaminoethylamino)methylen-3-(3,4,5-trimethoxyphenyl)-2-isoxazolin-5-on

4-Diethylaminomethylen-3-(3,4,5-trimethoxyphenyl)-2-isoxazolin-5-on

4-Morpholinomethylen-3-(3,4,5-trimethoxyphenyl)-2-isoxazolin-5-on

4-(4-Phenylpiperidin-1-yl)methylen-3-(3,4,5-trimethoxyphenyl)-2-isoxazolin 5-on

4-Perhydroazocin-1-ylmethylen-3-(3,4,5-trimethoxyphenyl)-2-isoxazolin-5-on

4-Piperidinomethylen-3-(4-nitrophenyl)-2-isoxazolin-5-on

4-(2-Dimethylaminoethylamino)methylen-3-(4-nitrophenyl)-2-isoxazolin-5-on

4-Diethylaminomethylen-3-(4-nitrophenyl)-2-isoxazolin-5-on

4-Morpholinomethylen-3-(4-nitrophenyl)-2-isoxazolin-5-on

4-(4-Phenylpiperidin-1-yl)methylen-3-(4-nitrophenyl)-2-isoxazolin-5-on

4-Perhydroazocin-1-ylmethylen-3-(4-nitrophenyl)-2-isoxazolin-5-on

4-Piperidinomethylen-3-(3-nitrophenyl)-2-isoxazolin-5-on

4-(2-Dimethylaminoethylamino)methylen-3-(3-nitrophenyl)-2-isoxazolin-5-on

4-Diethylaminomethylen-3-(3-nitrophenyl)-2-isoxazolin-5-on

4-Morpholinomethylen-3-(3-nitrophenyl)-2-isoxazolin-5-on

4-(4-Phenylpiperidin-1-yl)methylen-3-(3-nitrophenyl)-2-isoxazolin-5-on

4-Perhydroazocin-1-ylmethylen-3-(3-nitrophenyl)-2-isoxazolin-5-on

4-Piperidinomethylen-3-(2-nitrophenyl)-2-isoxazolin-5-on

4-(2-Dimethylaminoethylamino)methylen-3-(2-nitrophenyl)2-isoxazolin-5-on

4-Diethylaminomethylen-3-(2-nitrophenyl)-2-isoxazolin-5-on

4-Morpholinomethylen-3-(2-nitrophenyl)-2-isoxazolin-5-on

4-(4-Phenylpiperidin-1-yl)methylen-3-(2-nitrophenyl)-2-isoxazolin-5-on

4-Perhydroazocin-1-ylmethylen-3-(2-nitrophenyl)-2-isoxazolin-5-on

4-Piperidinomethylen-3-(4-methylphenyl)-2-isoxazolin-5-on

4-(2-Dimethylaminoethylamino)methylen-3-(4-methylphenyl)-2-isoxazolin-5-on

4-Diethylaminomethylen-3-(4-methylphenyl)-2-isoxazolin-5-on

4-Morpholinomethylen-3-(4-methylphenyl)-2-isoxazolin-5-on

4-(4-Phenylpiperidin-1-yl)methylen-3-(4-methylphenyl)-2-isoxazolin-5-on

4-Perhydroazocin-1-ylmethylen-3-(4-methylphenyl)-2-isoxazolin-5-on

4-Piperidinomethylen-3-(3-methylphenyl)-2-isoxazolin-5-on

4-(2-Dimethylaminoethylamino)methylen-3-(3-methylphenyl)-2-isoxazolin-5-on

4-Diethylaminomethylen-3-(3-methylphenyl)-2-isoxazolin-5-on

4-Morpholinomethylen-3-(3-methylphenyl)-2-isoxazolin-5-on

4-(4-Phenylpiperidin-1-yl)methylen-3-(3-methylphenyl)-2-isoxazolin-5-on

4-Perhydroazocin-1-ylmethylen-3-(3-methylphenyl)-2-isoxazolin-5-on

4-Piperidinomethylen-3-(2-methylphenyl)-2-isoxazolin-5-on

4-(2-Dimethylaminoethylamino)methylen-3-(2-methylphenyl)-2-isoxazolin-5-on

4-Diethylaminomethylen-3-(2-methylphenyl)-2-isoxazolin-5-on

4-Morpholinomethylen-3-(2-methylphenyl)-2-isoxazolin-5-on

4-(4-Phenylpiperidin-1-yl)methylen-3-(2-methylphenyl)-2-isoxazolin-5-on

4-Perhydroazocin-1-ylmethylen-3-(2-methylphenyl)-2-isoxazolin-5-on

4-Piperidinomethylen-3-(4-fluorphenyl)-2-isoxazolin-5-on

4-(2-Dimethylaminoethylamino)methylen-3-(4-fluorphenyl)-2-isoxazolin-5-on

4-Diethylaminomethylen-3-(4-fluorphenyl)-2-isoxazolin-5-on

4-Morpholinomethylen-3-(4-fluorphenyl)-2-isoxazolin-5-on

4-(4-Phenylpiperidin-1-yl)methylen-3-(4-fluorphenyl)-2-isoxazolin-5-on

4-Perhydroazocin-1-ylmethylen-3-(4-fluorphenyl)-2-isoxazolin-5-on

4-Piperidinomethylen-3-(3-fluorphenyl)-2-isoxazolin-5-on

4-(2-Dimethylaminoethylamino)methylen-3-(3-fluorphenyl)-2-isoxazolin-5-on

4-Diethylaminomethylen-3-(3-fluorphenyl)-2-isoxazolin-5-on

4-Morpholinomethylen-3-(3-fluorphenyl)-2-isoxazolin-5-on

4-(4-Phenylpiperidin-1-yl)methylen-3-(3-fluorphenyl)-2-isoxazolin-5-on

4-Perhydroazocin-1-ylmethylen-3-(3-fluorphenyl)-2-isoxazolin-5-on

4-Piperidinomethylen-3-(2-fluorphenyl)-2-isoxazolin-5-on

4-(2-Dimethylaminoethylamino)methylen-3-(2-fluorphenyl)-2-isoxazolin-5-on

4-Diethylaminomethylen-3-(2-fluorphenyl)-2-isoxazolin-5-on

4-Morpholinomethylen-3-(2-fluorphenyl)-2-isoxazolin-5-on

4-(4-Phenylpiperidin-1-yl)methylen-3-(2-fluorphenyl)-2-isoxazolin-5-on

4-Perhydroazocin-1-ylmethylen-3-(2-fluorphenyl)-2-isoxazolin-5-on

4-Piperidinomethylen-3-(4-isopropylphenyl)-2-isoxazolin-5-on

4-(2-Dimethylaminoethylamino)methylen-3-(4-isopropylphenyl)-2-isoxazolin-5-on

4-Diethylaminomethylen-3-(4-isopropylphenyl)-2-isoxazolin-5-on

4-Morpholinomethylen-3-(4-isopropylphenyl)-2-isoxazolin-5-on

4-(4-Phenylpiperidin-1-yl)methylen-3-(4-isopropylphenyl)-2-isoxazolin-5-on

4-Perhydroazocin-1-ylmethylen-3-(4-isopropylphenyl)-2-isoxazolin-5-on

4-(1-Perhydroazocin-1-ylethyliden)-3-(2-fluorphenyl)-2-isoxazolin-5-on

4-[1-(4-Phenylpiperidin-1-yl)-ethyliden]-3-(3-methoxyphenyl)-2-isoxazolin-5-on

4-(1-Piperidinoethyliden)-3-(4-methylphenyl)-2-isoxazolin 5-on

Die Verbindungen der Formel I besitzen wertvolle pharmakologische Eigenschaften, insbesondere nootrope. Die nootrope Wirkung der erfindungsgemäßen Verbindungen wurde an Mäusen, die ein Körpergewicht von 20 -25 g besaßen, im inhibitory (passive) avoidance test geprüft. Eine modifizierte Form der von J. Kopp, Z. Bodanecky und M.E. Jarvik beschriebenen Testmethode wurde von J. Bures, O. Buresova und J. Huston in "Techniques and Basic Experiments for the Study of Brain and Behavior", Elsevier Scientific Publishers, Amsterdam 1983, beschrieben.

Entsprechend diesen Literaturangaben wird eine Substanz dann als nootrop wirksam bezeichnet, wenn sie bei den Versuchstieren die mittels eines elektroconvulsiven Schocks erzeugte Amnesie aufzuheben vermag.

Die Versuche wurden nach modifizierten Testmethoden mit den erfindungsgemäßen Verbindungen der Beispiele 3 und 13 durchgeführt. Als Vergleichsverbindung diente das bekannte Nootropikum 2-Oxo-1-pyrrolidinylessigsäureamid - (Piracetam). Die deutliche Überlegenheit der erfindungsgemäßen Verbindungen über die Vergleichssubstanz zeigte sich darin, daß der amnestische Effekt bereits mit einer MED (minimal effektive Dosis) von 0,1 -10 mg/kg p.o. aufzuheben ist. Die Vergleichssubstanz hat eine MED von ca. 500 - 1000 mg/kg p.o.

Die Scopolamin-induzierte Amnesie im inhibitory avoidance test läßt sich mit einer MED von 1,0 -20 mg/kg p.o. (Verbindung gemäß Beispiel 3) aufheben. In diesem Test wurde für die Verbindungen entsprechend den Beispielen 20,37,38,42,44 und 49 eine MED von 6-25 mg/kg p.o. ermittelt.

Die erfindungsgemäßen Verbindungen haben nur geringe Toxizität; die oralen $LD_{50}$-Werte an Mäusen liegen über 500 mg/kg.

Die erfindungsgemäßen Verbindungen sind aufgrund ihrer pharmakologischen Eigenschaften für die Behandlung cognitiver Dysfunktionen unterschiedlicher Genese, wie sie z.B. bei der Alzheimerschen Krankheit oder der senilen Demenz auftreten, geeignet.

Die Erfindung betrifft daher weiter die Anwendung der erfindungsgemäßen Verbindungen der Formel I bei der Behandlung und Prophylaxe cognitiver Dysfunktionen.

Die Erfindung umfaßt weiterhin die Verwendung der erfindungsgemäßen Verbindungen bei der Herstellung von Arzneimitteln, die zur Behandlung und Prophylaxe der vorstehend genannten Krankheiten eingesetzt werden.

Ein weiterer Gegenstand der Erfindung sind Arzneimittel, die ein oder mehrere erfindungsgemäße Verbindungen der allgemeinen Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche, oder vorzugsweise in Kombination mit geeigneten pharmazeutischen Hilfsstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 %, beträgt.

Welche Hilfsstoffe für die gewünschte Arzneimittelformulierung geeignet sind, ist dem Fachmann aufgrund seines Fachwissens geläufig. Neben Lösemitteln, Gelbildnern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Die Wirkstoffe können oral, parenteral, intravenös oder rektal appliziert werden, wobei die orale Applikation bevorzugt ist. Eine Dosierungseinheit für die orale Applikation enthält z.B. 1,0 bis 1000 mg, vorzugsweise 50 bis 500 mg Wirkstoff, jedoch können auch darüber oder darunter liegende Dosierungen verwendet werden.

Für eine orale Anwendungsform werden die aktiven Verbindungen mit den dafür geeigneten Zusatzstoffen wie Trägerstoffen, Stabilisatoren oder inerten Verdünnungsmitteln vermischt und durch die üblichen Methoden in geeignete Darreichungsformen gebracht, wie Tabletten, Dragees, Steckkapseln, wäßrige alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose oder Stärke, insbesondere Maisstärke verwendet werden. Dabei kann die Zubereitung sowohl als Trocken-als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation werden die aktiven Verbindungen oder deren physiologisch verträg lichen Salze, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage Wasser, physiologische Kochsalzlösung oder Alkohole, z.B. Ethanol, Propanol, Glycerin, daneben auch Zuckerlösungen wie Glucose-oder Mannitlösungen, oder auch eine Mischung aus den verschiedenen genannten Lösungsmitteln.

Beispiel 1

4-Morpholinomethylen-3-phenyl-2-isoxazolin-5-on

Eine Suspension von 9,67 g (60 mMol) 3-Phenyl-2-isoxazolin-5-on in 180 ml trockenem Chloroform wird mit 20,91 g (mMol) Morpholin versetzt. In die entstandene klare Lösung wird bis zum Reaktionsende ein ständiger Stickstoffstrom eingeleitet. Nach Zugabe von 1,78 g (22 mMol) 1,3,5-Triazin wird die Reaktionslösung zum Sieden erhitzt, bis am Ende des Rückflußkühlers kein Ammoniak mehr nachzuweisen ist, (ca. 3 h). Anschließend wird das Reaktionsgemisch zur Vermehrung des entstandenen Niederschlags im Eisbad gekühlt; das Rohprodukt wird abgesaugt, mehrmals mit eiskaltem Diethylether gewaschen und aus Methanol umkristallisiert. Schwach rosafarbene Plättchen vom Schmp. 193 °C

Analog zu Beispiel 1 wurden die folgenden Verbindungen hergestellt (Tabelle 1):

Tabelle 1

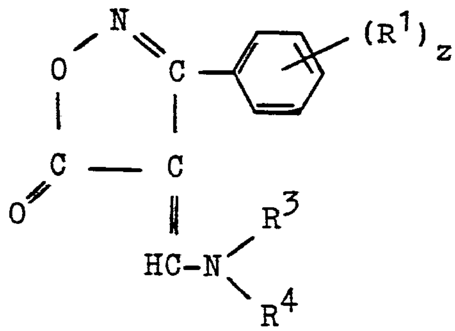

| Beispiel | $(R^1)_z$ | 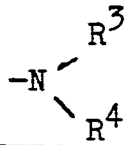 | t | Schmp. |
|---|---|---|---|---|
| 2 | H | 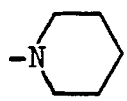 | 72 h | 182 °C |
| 3 | H | 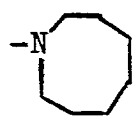 | 30 h | 176 °C |
| 4 | H | 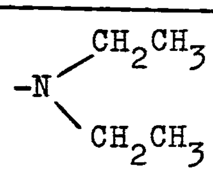 | 17 h | 68 °C |
| 5 | H | 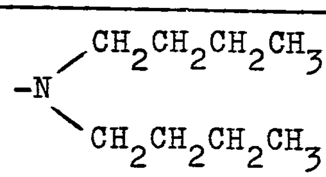 | 56 h | 87 °C |
| 6 | H | 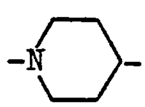 $-CO_2C_2H_5$ | 6 h | 105 °C |

Tabelle 1 (Fortsetzung)

| Beispiel | $(R^1)_z$ | 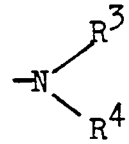 | t | Schmp. |
|---|---|---|---|---|
| 7 | H | 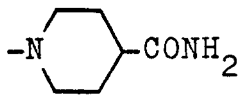 | 11 h | 181 °C |
| 8 | H | 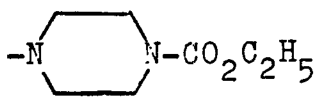 | 1,5 h | 194 °C |
| 9 | H | 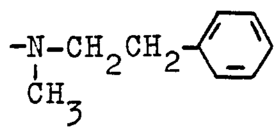 | 18 h | 171 °C |
| 10 | H | 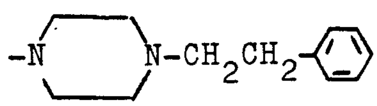 | 6 h | 179 °C |
| 11 | H | 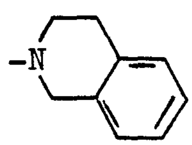 | 6 h | 141 °C |
| 12 | H | 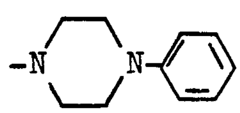 | 2 h | 177 °C |
| 13 | H | 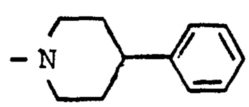 | 30 h | 204 °C |
| 14 | H | 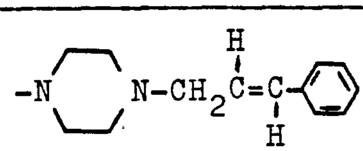 | 7 h | 176 °C |

Tabelle 1 (Fortsetzung)

| Beispiel | $(R^1)_z$ | 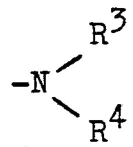 | t | Schmp. |
|---|---|---|---|---|
| 15 | H | 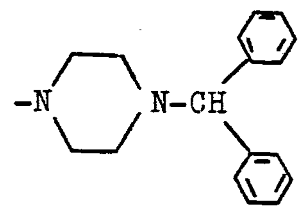 | 5,5 h | 220 °C |
| 16 | H | 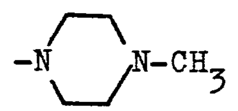 | 2 h | 163 °C |
| 17 | H | 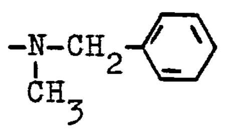 | 8 h | 146 °C |

t = Reaktionszeit

Beispiel 18

4-Ethylaminomethylen-3-phenyl-2-isoxazolin-5-on

0,44 g (2,1 mmol) des Natriumsalzes des 4-Hydroxymethylen-3-phenyl-2-isoxazolin-5-ons (W. Müller et al., Tetrahedron 29(24), 4291-8 (1973)) gelöst in 10 ml Wasser werden mit 20 proz. Essigsäure leicht angesäuert und sofort mit 5 ml einer 70 proz. wäßrigen Ethylaminlösung versetzt. 15 minütiges Erhitzen unter Rückfluß und anschließende Kühlung auf 4 °C liefert ein Rohprodukt, das abgesaugt, mit wenig kaltem Wasser gewaschen und aus Isopropanol umkristallisiert wird. Farblose Nadeln vom Schmp. 150°C.

Beispiel 19

3-(4-Chlorphenyl)-4-(piperidinomethylen)-2-isoxazolin-5-on

a) 59 g (0.26 Mol) p-Chlorbenzoylessigsäureethylester werden in 310 ml Ethanol gelöst. Dazu gibt man eine konzentrierte wäßrige Lösung von 18.7 g Hydroxylaminhydrochlorid und erhitzt die Mischung 15 Minuten auf dem Dampfbad. Durch Kühlen im Eisbad kristallisiert das gebildete 3-(4-Chlorphenyl)-2-isoxazolin-5-on aus. Es wird abgesaugt und mit wenig kaltem Ethanol nachgewaschen.

Schmelzpunkt 58°C.

b) ·20 g (0.1 Mol) 3-(4-Chlorphenyl)-2-isoxazolin-5-on werden in 500 ml Trimethylorthoformiat gelöst und 4 Tage bei Raumtemperatur unter Feuchtigkeitsausschluß stehengelassen. Man destilliert das Lösungsmittel im Vakuum ab und kristallisiert den Rückstand aus Ethanol um. Man erhält 3-(4-Chlorphenyl)-4-

(methoxymethylen)-2-isoxazolin-5-on gelblichen Feststoff vom Schmelzpunkt 115°C.

c) 2 g (8.4 mMol) 3-(4-Chlorphenyl)-4-(methoxymethylen)-2-isoxazolin-5-on werden in 10 ml Tetrahydrofuran gelöst und mit 1.7 ml (17 mMol) Piperidin versetzt. Man rührt 20 h bei Raumtemperatur, destilliert das Lösungsmittel im Vakuum ab und kristallisiert den Rückstand aus Ethanol um. Leicht gelbliche Kristalle

Schmelzpunkt 182°C.

Analog zu Beispiel 19 werden die in Tabelle 2 ausgeführten Verbindungen erhalten.

## Tabelle 2

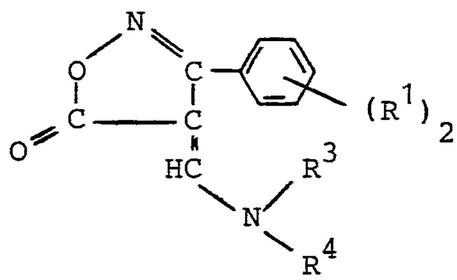

| Beispiel | $(R^1)_z$ | 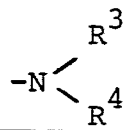 | Schmelzpunkt |
|---|---|---|---|
| 20 | H | 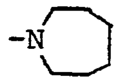 | 145°C |
| 21 | H | 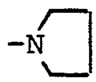 | 172°C |
| 22 | H | 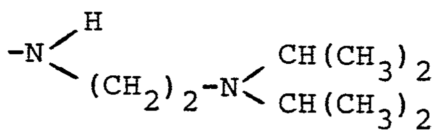 | 125°C |
| 23 | 4-Cl | 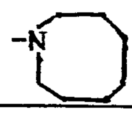 | 167°C |
| 24 | 4-Cl | 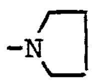 | 148°C |
| 25 | 4-Cl | 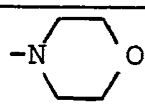 | 192°C |
| 26 | 4-Cl | 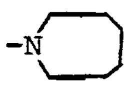 | 159°C |
| 27 | H | 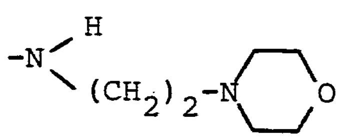 | 148°C |
| 28 | H | 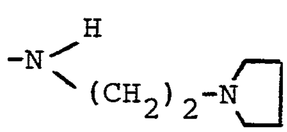 | 139°C |

Tabelle 2 (Fortsetzung)

| Beispiel | $(R^1)_z$ | 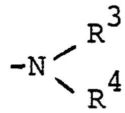 | Schmelzpunkt |
|---|---|---|---|
| 29 | H | 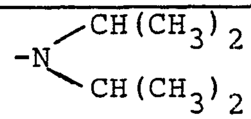 | 179°C |
| 30 | 4-Cl | 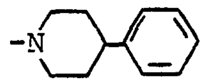 | 177°C |
| 31 | H | 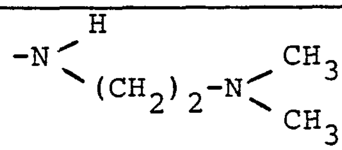 | 103°C |
| 32 | H | 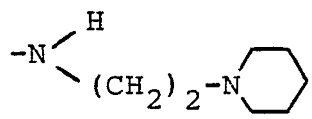 | 111°C |
| 33 | H | 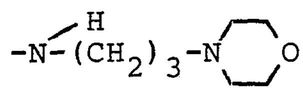 | 101°C |
| 34 | 4-Cl | 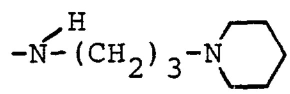 | 137°C |
| 35 | 4-Cl | 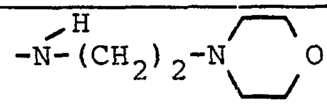 | 185°C |
| 36 | H | 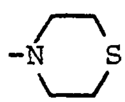 | 201°C |
| 37 | 4-Cl | 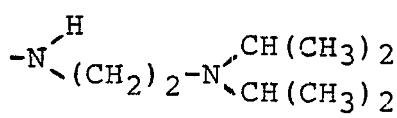 | 134°C |
| 38 | H | 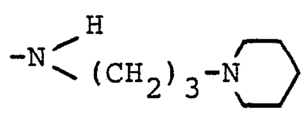 | 101°C |
| 39 | 2-OMe | 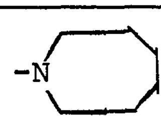 | 202°C |
| 40 | 4-Cl | 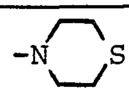 | 204°C |

Tabelle 2 (Fortsetzung)

| Beispiel | $(R^1)_z$ | 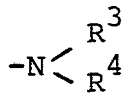 | Schmelzpunkt |
|---|---|---|---|
| 41 | 4-Cl | 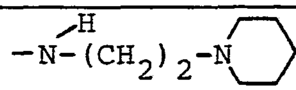 | 162°C |
| 42 | 4-Cl | 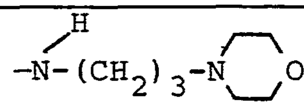 | 164°C |
| 43 | 4-Cl | 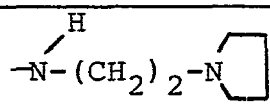 | 153°C |
| 44 | H | 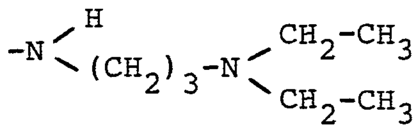 | 97°C |
| 45 | H | 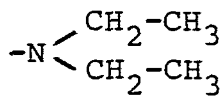 | 76-78°C |
| 46 | 4-Cl | 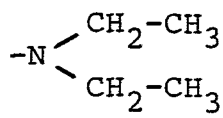 | 128°C |
| 47 | 4-Cl | 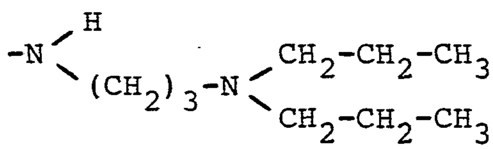 | 93°C |
| 48 | 4-Cl | 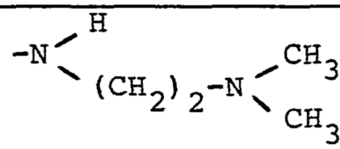 | 136°C |
| 49 | 2-Cl | 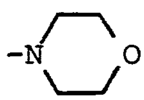 | 150°C |
| 50 | 4-Cl | 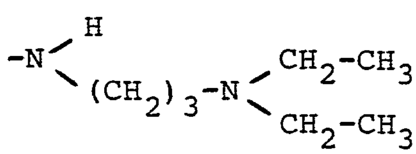 | 91°C |
| 51 | 4-Cl | 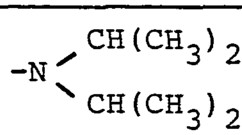 | 105°C |

Tabelle 2 (Fortsetzung)

| Beispiel | $(R^1)_z$ | $-N\genfrac{}{}{0pt}{}{R^3}{R^4}$ | Schmelzpunkt |
|---|---|---|---|
| 52 | 4-Cl | $-N\genfrac{}{}{0pt}{}{CH_3}{CH_3}$ | 127°C |
| 53 | H | $-N\genfrac{}{}{0pt}{}{H}{(CH_2)_4-N\genfrac{}{}{0pt}{}{CH_3}{CH_3}}$ | 122°C |

Beispiel 54

4-[N-(3-Dimethylaminopropyl)aminomethylen]-3-phenyl-2-isoxazolin-5-on Hydrochlorid

2 g (12.4 mMol) 4-(Methoxymethylen)-2-phenyl-2-isoxazolin-5-on werden in 20 ml Tetrahydrofuran gelöst und tropfenweise unter Eiskühlung mit 1.5 ml (12.4 mMol) 3-Dimethylaminopropylamin versetzt. Nach Abklingen der exothermen Reaktion wird noch 4 h bei Raumtemperatur gerührt und dann das Lösungsmittel im Vakuum abdestilliert. Das zurückbleibende Öl wird in 25 ml Isopropanol gelöst und mit 3 ml 3.8N HCl in Isopropanol versetzt. Der gebildete Niederschlag wird abgesaugt und aus Ethanol umkristallisiert.

Schmelzpunkt 206-8°C

Beispiel 55

4-[N-(2-Morpholinoethyl)aminomethylen]-3-phenyl-2-isoxazolin-5-on

1.6 g (10 mMol) 3-Phenyl-2-isoxazolin-5-on und 1.3g (10 mMol) 2-Morpholinoethylamin werden in 10 ml Trimethylorthoformiat 1 h unter Rückfluß gekocht. Das Lösungsmittel wird im Vakuum abdestilliert und der Rückstand mehrfach aus Isopropanol umkristallisiert.

Schmelzpunkt 148°C, identisch mit Beispiel 27

Beispiel 56

3-(4-Chlorphenyl)-4-(piperidinomethylen)-2-isoxazolin-5-on

1.74 g (6.94 mMol) 3-(4-Chlorphenyl)-4-(dimethylaminomethylen)-2-oxoazolin-5-on werden mit 1.4 ml Piperidin und 20 ml Toluol 6 h zum Sieden erhitzt. Das Lösungsmittel wird im Vakuum abdestilliert und der Rückstand aus Ethanol umkristallisiert.

Schmelzpunkt 182°C, identisch mit Beispiel 19

**Ansprüche**

1. 5(4H)-Isoxazolone der allgemeinen Formel I

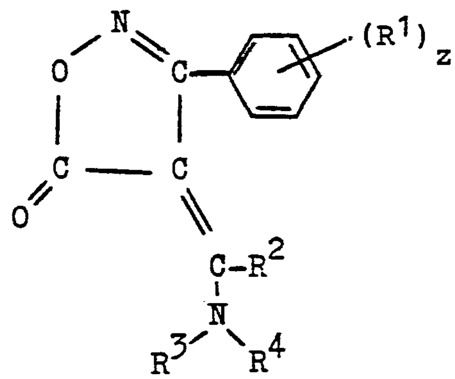

(I)

worin

$R^1$ Wasserstoff, $C_1$-$C_4$-Alkyl, $C_1$-$C_4$-Alkoxy, Hydroxy, Halogen, Nitro oder Trifluormethyl ist,

z für 1, 2 oder 3 steht,

$R^2$ Wasserstoff oder $C_1$-$C_4$-Alkyl bedeutet,

$R^3$ Wasserstoff oder $C_1$-$C_4$-Alkyl und

$R^4$ Wasserstoff, $C_1$-$C_{10}$-Alkyl oder eine Gruppe der Formel $-(CH_2)_n-R^5$ ist, worin n = 1 bis 10 ist und $R^5$ ein Phenylrest, der mit $C_1$-$C_4$-Alkyl, $C_1$-$C_4$-Alkoxy, Halogen, Nitro oder Trifluormethyl substituiert sein kann, oder ein Aminorest der Formel

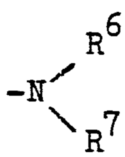

ist, worin $R^6$ Wasserstoff oder $C_1$-$C_4$-Alkyl und $R^7$ $C_1$-$C_4$-Alkyl oder Phenyl-($C_1$-$C_4$)alkyl bedeutet oder worin $R^6$ und $R^7$ zusammen eine Alkylenkette mit 3 bis 7 Methylengruppen darstellen, die gegebenenfalls substituiert ist mit $C_1$-$C_4$-Alkyl oder Phenyl, das gegebenenfalls substituiert ist, mit $C_1$-$C_4$-Alkyl, $C_1$-$C_4$-Alkoxy, Halogen, Nitro oder Trifluormethyl, oder in der eine $CH_2$-Gruppe gegebenenfalls ersetzt ist durch O, S oder N-$R^8$, wobei $R^8$ Wasserstoff, $C_1$-$C_4$-Alkyl oder Phenyl, das gegebenenfalls mit $C_1$-$C_4$-Alkyl, $C_1$-$C_4$-Alkoxy, Halogen , Nitro oder Trifluormethyl substituiert ist, darstellt oder

$R^3$ und $R^4$ bilden zusammen mit dem Stickstoffatom einen gesättigten oder ungesättigten mono- oder bicyclischen Ring mit insgesamt 4 bis 10 Ringgliedern, der außer dem Stickstoffatom als zweites Heteroatom ein Sauerstoff-, Schwefel-oder Stickstoffatom aufweisen kann und der gegebenenfalls substituiert ist mit $C_1$-$C_4$-Alkyl, mit $C_1$-$C_4$-Alkoxycarbonyl, mit Carbamoyl, mit Cinnamyl, mit Phenyl, das mit Halogen, $C_1$-$C_4$-Alkyl, $C_1$-$C_4$-Alkoxy, oder Trifluormethyl substituiert sein kann, oder mit der Gruppe $-(CH_2)_y-CH_{3-m}R^9{}_m$, worin y = 0 bis 3, m 1 oder 2 und $R^9$ ein Phenylrest ist, der durch Halogen, $C_1$-$C_4$-Alkyl, $C_1$-$C_4$-Alkoxy oder Trifluormethyl substituiert sein kann,

wobei Verbindungen, worin $R^1$ und $R^2$ Wasserstoff und $R^3$ und $R^4$ Methyl bedeuten, ausgenommen sind, und deren physiologisch verträgliche Säureadditionssalze.

2. 5(4H)-Isoxazolone gemäß Anspruch 1, dadurch gekennzeichnet, daß in Formel I $R^1$ Wasserstoff oder Chlor und $R^2$ Wasserstoff bedeuten und $R^3$ und $R^4$ zusammen mit dem Stickstoffatom einen gesättigten, monocyclischen Ring mit 6 bis 8 Ringgliedern bilden, wobei dieser Ring gegebenenfalls mit Phenyl substituiert ist.

3. 4-Perhydroazocin-1-ylmethylen-3-phenyl-2-isoxazolin-5-on gemäß Anspruch 1 sowie physiologisch verträgliche Säureadditionssalze.

4. 4-(4-Phenylpiperidin-1-yl)methylen-3-phenyl-2-isoxazolin-5-on gemäß Anspruch 1 sowie physiologisch verträgliche Säureadditionssalze.

5. Verfahren zur Herstellung von 5(4H)-Isoxazolonen und deren Salzen gemäß Anspruch 1, dadurch gekennzeichnet, daß man

a) eine Verbindung der allgemeinen Formel II

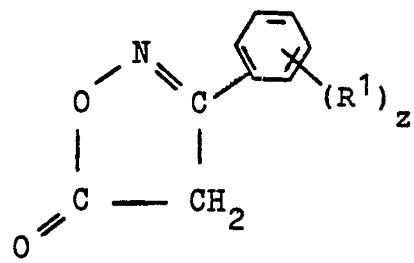

(II)

und ein Amin der Formel III

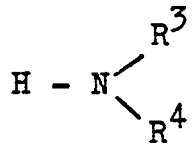

(III)

worin $R^1$, $R^3$, $R^4$ und z die gleichen Bedeutungen wie in Formel I haben, mit 1,3,5-Triazin zu einer Verbindung der Formel I, worin $R^2$ Wasserstoff ist und $R^1$, $R^3$, $R^4$ und z die angegebenen Bedeutungen haben, umsetzt;

b) eine Verbindung der allgemeinen Formel II und ein Amin der allgemeinen Formel III, worin z, $R^1$, $R^3$ und $R^4$ die gleichen Bedeutungen wie in Formel I haben, mit einem Orthoester der allgemeinen Formel IV,

$R^2C(OR^{10})_3$ (IV)

worin $R^2$ die oben genannte Bedeutung hat und $R^{10}$ Methyl oder Ethyl ist, umsetzt; oder

c) eine Verbindung der allgemeinen Formel V, worin z, $R^1$ und $R^2$

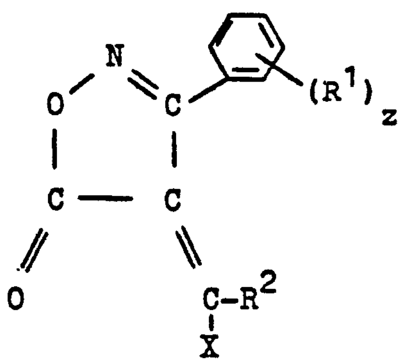

(V)

die unter Formel I aufgeführte Bedeutung haben und X eine Hydroxy-, Methoxy-, Ethoxy-, Amino, $C_1$-$C_4$-Alkylamino, $C_1$-$C_4$-Dialkylamino-oder Anilinogruppe ist, mit einem Amin der allgemeinen Formel III umsetzt,

und die Reaktionsprodukte gegebenenfalls in physiologisch verträgliche Säureadditionssalze überführt.

6. Pharmazeutische Zubereitungen, dadurch gekennzeichnet, daß sie eine Verbindung gemäß Anspruch 1 und einen pharmazeutisch verträglichen Träger enthalten.

7. Verfahren zur Herstellung einer pharmazeutischen Zubereitung dadurch gekennzeichnet, daß man eine Verbindung gemäß Anspruch 1 in eine geeignete Darreichungsform überführt.

8. Verwendung einer Verbindung gemäß Anspruch 1 zur Behandlung cognitiver Dysfunktionen.

9. Verfahren zur Behandlung cognitiver Dysfunktionen durch Applikation einer wirksamen Menge einer Verbindung gemäß Anspruch 1.

Patentansprüche für dem Vertragsstaat : AT

1. Verfahren zur Herstellung von 5(4H)-Isoxazolonen der allgemeinen Formel I

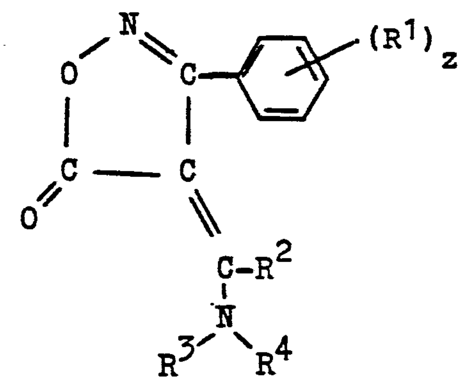

(I)

worin

$R^1$ Wasserstoff, $C_1$-$C_4$-Alkyl, $C_1$-$C_4$-Alkoxy, Hydroxy, Halogen, Nitro oder Trifluormethyl ist,

z für 1, 2 oder 3 steht,

$R^2$ Wasserstoff oder $C_1$-$C_4$-Alkyl bedeutet,

$R^3$ Wasserstoff oder $C_1$-$C_4$-Alkyl und

$R^4$ Wasserstoff, $C_1$-$C_{10}$-Alkyl oder eine Gruppe der Formel $-(CH_2)_n-R^5$ ist, worin n = 1 bis 10 ist und $R^5$ ein Phenylrest, der mit $C_1$-$C_4$-Alkyl, $C_1$-$C_4$-Alkoxy, Halogen, Nitro oder Trifluormethyl substituiert sein kann, oder ein Aminorest der Formel

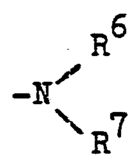

ist, worin $R^6$ Wasserstoff oder $C_1$-$C_4$-Alkyl und $R^7$ $C_1$-$C_4$-Alkyl oder Phenyl-($C_1$-$C_4$)alkyl bedeutet oder worin $R^6$ und $R^7$ zusammen eine Alkylenkette mit 3 bis 7 Methylengruppen darstellen, die gegebenenfalls substituiert ist mit $C_1$-$C_4$-Alkyl oder Phenyl, das gegebenenfalls substituiert ist, mit $C_1$-$C_4$-Alkyl, $C_1$-$C_4$-Alkoxy, Halogen, Nitro oder Trifluormethyl, oder in der eine $CH_2$-Gruppe gegebenenfalls ersetzt ist durch O, S oder N-$R^8$, wobei $R^8$ Wasserstoff, $C_1$-$C_4$-Alkyl oder Phenyl, das gegebenenfalls mit $C_1$-$C_4$-Alkyl, $C_1$-$C_4$-Alkoxy, Halogen , Nitro oder Trifluormethyl substituiert ist, darstellt oder

$R^3$ und $R^4$ bilden zusammen mit dem Stickstoffatom einen gesättigten oder ungesättigten mono- oder bicyclischen Ring mit insgesamt 4 bis 10 Ringgliedern, der außer dem Stickstoffatom als zweites Heteroatom ein Sauerstoff-, Schwefel-oder Stickstoffatom aufweisen kann und der gegebenenfalls substituiert ist mit $C_1$-$C_4$-Alkyl, mit $C_1$-$C_4$-Alkoxycarbonyl, mit Carbamoyl, mit Cinnamyl, mit Phenyl, das mit Halogen, $C_1$-$C_4$-Alkyl, $C_1$-$C_4$-Alkoxy, oder Trifluormethyl substituiert sein kann, oder mit der Gruppe $-(CH_2)_y-CH_{3-m}R^9m$, worin y = 0 bis 3, m 1 oder 2 und $R^9$ ein Phenylrest ist, der durch Halogen, $C_1$-$C_4$-Alkyl, $C_1$-$C_4$-Alkoxy oder Trifluormethyl substituiert sein kann,

wobei Verbindungen, worin $R^1$ und $R^2$ Wasserstoff und $R^3$ und $R^4$ Methyl bedeuten, ausgenommen sind, und von deren physiologisch verträglichen Säureadditionssalzen, dadurch gekennzeichnet, daß man

a) eine Verbindung der allgemeinen Formel II

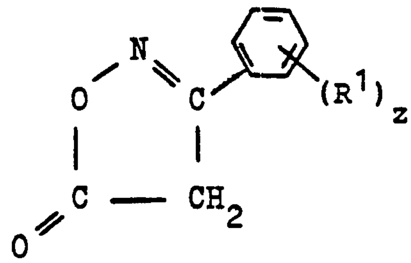

(II)

und ein Amin der Formel III

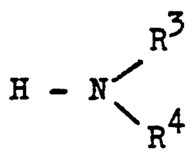

(III)

worin $R^1$, $R^3$, $R^4$ und z die gleichen Bedeutungen wie in Formel I haben, mit 1,3,5-Triazin zu einer Verbindung der Formel I, worin $R^2$ Wasserstoff ist und $R^1$, $R^3$, $R^4$ und z die angegebenen Bedeutungen haben, umsetzt;

b) eine Verbindung der allgemeinen Formel II und ein Amin der allgemeinen Formel III, worin z, $R^1$, $R^3$ und $R^4$ die gleichen Bedeutungen wie in Formel I haben, mit einem Orthoester der allgemeinen Formel IV,

$R^2C(OR^{10})_3$ (IV)

worin $R^2$ die oben genannte Bedeutung hat und $R^{10}$ Methyl oder Ethyl ist, umsetzt; oder

c) eine Verbindung der allgemeinen Formel V, worin z, $R^1$ und $R^2$

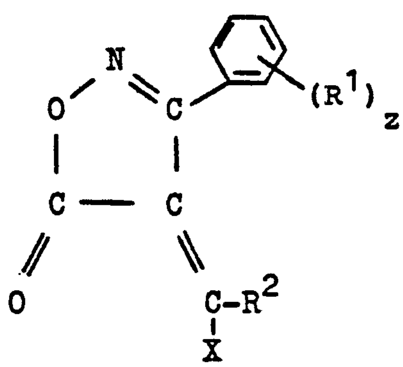

(V)

die unter Formel I aufgeführte Bedeutung haben und X eine Hydroxy-, Methoxy-, Ethyoxy-, Amino, $C_1$-$C_4$-Alkylamino, $C_1$-$C_4$-Dialkylamino- oder Anilinogruppe ist, mit einem Amin der allgemeinen Formel III umsetzt,

und die Reaktionsprodukte gegebenenfalls in physiologisch verträgliche Säureadditionssalze überführt.

2. Verfahren gemäß Anspruch 1, dadurch gekennzeichnet, daß man Verbindungen der Formel I, worin $R^1$ Wasserstoff oder Chlor und $R^2$ Wasserstoff bedeuten und $R^3$ und $R^4$ zusammen mit dem Stickstoff atom einen gesättigten, monocyclischen Ring mit 6 bis 8 Ringgliedern bilden, wobei dieser Ring gegebenenfalls mit Phenyl substituiert ist, oder deren Säureadditionssalze herstellt.

3. Verfahren gemäß Anspruch 1, dadurch gekennzeichnet, daß man 4-Perhydroazocin-1-ylmethylen-3-phenyl-2-isoxazolin-5-on oder ein physiologisch verträgliches Salz herstellt.

4. Verfahren gemäß Anspruch 1, dadurch gekennzeichnet, daß man 4-(4-Phenylpiperidin-1-yl)methylen-3-phenyl-2-isoxazolin-5-on oder ein

physiologisch veträgliches Salz herstellt.

5. Verfahren zur Herstellung einer pharmazeutischen Zubereitung dadurch gekennzeichnet, daß man eine Verbindung der Formel I gemäß Anspruch 1 in eine geeignete Darreichungsform überführt.

6. 5(4H)-Isoxazolone der Formel I

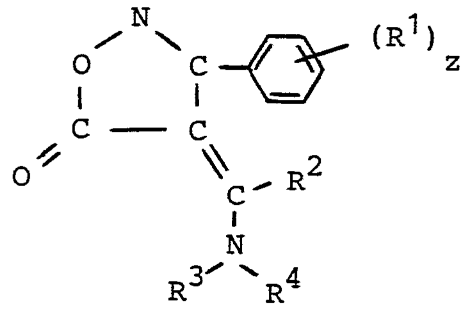

I

worin $R^1$, $R^2$, $R^3$, $R^4$ und z die in Anspruch 1 zu Formel I angegebenen Bedeu·ungen haben, erhältlich nach Verfahren gemäß Anspruch 1.